# EUROPEAN PATENT APPLICATION

(11) **EP 4 199 001 A1**
(43) Date of publication of application: **21.06.2023**
(21) Application number: 21383136.5
(22) Date of filing: 14.12.2021
(51) Int. Cl.: G16H 40/60, G16H 40/00

(54) **SYSTEM AND METHOD FOR MONITORING PERFORMANCE OF A MEDICAL DEVICE**

(71) Applicant: F. Hoffmann-La Roche AG, 4070 Basel (CH); Roche Diagnostics GmbH, 68305 Mannheim (DE)
(72) Inventor: MORCILLO MONTEJO, Alejandro, Barcelona, Saint Cugat del Vallès 08164, (ES)
(74) Representative: Mewburn Ellis LLP

(57) **Abstract**

A medical device monitoring system, method, and engine. The system comprises: a data processing module, configured to receive and process data related to one or more processes associated with one or more medical devices; and a user terminal, configured to display one or more tracked performance metrics associated with the or each process, and to receive, from a user, a selected time in a history of the tracked performance metric and to provide the selected time to the data processing module; wherein the data processing module is configured, in response to receiving the selected time, to: derive from the received and processed data at least one factor which has contributed to the value of the tracked performance metric at the selected time, the or each factor comprising an event which has taken place with respect to the respective process, and output to the user, via the user terminal, the or each derived factor.

## Description

### Field of the Disclosure

The present disclosure relates to a medical device monitoring system, a computer-implemented method of monitoring a medical device, and a medical device monitoring engine.

### Background

It is important in systems using medical devices that operators and/or technicians are appraised of the performance of these systems. For example, in vitro diagnostic testing has a major effect on clinical decisions, providing physicians with pivotal information.

Conventionally, it may not be possible for a lab manger to know what has happened which ultimately affected a key performance metric (e.g. sample turnaround time, or TAT, which can be understood as a metric identifying how long it takes for a result to be returned after a test has been requested). It could be something relatively obvious, e.g. a laboratory instrument being down for maintenance. However, it could also be that something was changed which has an indirect affect on the key performance metric. This can be particularly important, for example, where samples must be processed 'STAT' (i.e. as fast as possible). Such samples are typically required to make urgent clinical decisions, for example whether to continue excising potentially cancerous tissue during surgery or which specific antibiotic to prescribe for a yet-to-be-identified bacterial infection.

The disclosure was devised in light of the above considerations.

### Summary

In a first aspect, embodiments of the disclosure provide a medical device monitoring system comprising:
a data processing module, configured to receive and process data related to one or more processes associated with one or more medical devices; and
a user terminal, configured to display one or more tracked performance metrics associated with the or each process, and to receive, from a user, a selected time in a history of the tracked performance metric and to provide the selected time to the data processing module;
wherein the data processing module is configured, in response to receiving the selected time, to:
   derive from the received and processed data at least one factor which has contributed to the value of the tracked performance metric at the selected time, the or each factor comprising an event which has taken place with respect to the respective process, and
   output, to the user, via the user terminal, the or each derived factor.

Such a monitoring system improves the utilisation rate and efficiency of medical devices as factors causing degradation in performance can be identified and mitigated against.

The medical device monitoring system may be, for example, a laboratory device monitoring system or a point-of-care device monitoring system. The medical device monitoring system may be implemented as software or a suite of software running on one or more devices. The medical device monitoring system may be operating on the user terminal itself. The data processing module may be a laboratory management module or it may be a point-of-care management module. These modules may be provided as software or a suite of software running on one or more devices. These modules may be provided as dedicated elements of computer hardware. The user terminal may be one of: a smart phone, a tablet, a laptop computer, or a desktop computer. The derived factor may include an identifier of an event which has taken place during or with respect to the process. By factor, it may be meant a medical device status, medical device operation parameter, or medical device utilisation indicator related to a given one or more medical device(s). The medical device status, medical device parameter, or medical device utilisation indicator may have been identified as influencing the tracked performance metric. The medical device status may indicate, for example, that the medical device is unavailable for use or has a particular fault. The medical device operation parameter may indicate, for example, that the medical device is operating in a particular mode or using a particular consumable. The medical device utilisation indicator may indicate, for example, a medical device uptime or downtime or whether a medical device is available for use or is presently being used. For example the factor may identify that a reagent ran out during processing of a biological sample or that a point-of-care device underwent a software or firmware update.

The data may relate to performance of one or more of: a work area in a laboratory; a testing process performed in a laboratory; a point-of-care device; and a laboratory instrument used in a laboratory. The data may be indicative of a plurality of events occurring with respect to the one or more medical devices and/or data indicative of one or more environmental parameters associated with the one or more medical devices.

One of the data processing module and the user terminal may be configured to provide alerts to the user when the tracked performance metric deviates from a predefined acceptable value range. The provision of alerts can help ensure that issues with the medical device(s) are identified and mitigated quickly and so minimise the impact on performance. The alert may be an audio and/or visual alert. The predefined acceptable value range may be user defined, or may be derived by the system using historical data (e.g. a range of previously known to be accepted values). The predefined acceptable value range may be arrived at using an analysis model which predicts what an expected value of the tracked performance metric should be and compares it to the corresponding actual tracked performance metric.

The medical device monitoring system may further comprise a prediction module configured to predict an impact a user specified factor has on the tracked performance metric. For example, a user may consider modifying a setting on the one or more medical devices, and the prediction module will then predict the impact modifying this setting has on the tracked performance metric.

The tracked performance metric may be one of: a sample turnaround time, a late sample tracking value, a connection status, sample workload, laboratory instrument throughput, reagent capacity, a quality control value, and a point-of-care device uptime.

The user terminal may be configured to receive, from the user, a definition of a performance metric to be tracked, and the data processing module may be configured to then receive and process data relating to the defined performance metric. This can increase the flexibility of the medical device monitoring system, as the user can specify performance metrics of importance to them.

The user terminal may be configured to display to the user a benchmark performance metric associated with the or each process for comparison with the tracked performance metric. The benchmark performance metric may be defined, or may be derived using data from a plurality of medical device monitoring systems.

Deriving the at least one factor may include using an analysis model which relates data pertaining to the process to a value of the performance metric. The analysis model may be a machine-learning model, and may have been trained by, in an initial training phase:
(a) receiving input data comprising one or more of:
   data indicative of a plurality of events occurring with respect to the one or more medical devices; and
   data indicative of one or more environmental parameters associated with the one or more medical devices;
(b) receiving data indicative of the tracked performance metric; and
(c) training the machine-learning model using the input data as an input and the data indicative of the performance of the tracked performance metric as a desired output,
whereby the trained machine-learning model is able to relate new input data to the tracked performance metric.

Steps (a) - (c) may have been performed on historical input data and historical data indicative of the tracked performance metric; and the analysis model may be continuously retrained by:
(d) providing additional input data to the analysis model comprising one or more of:
   data indicative of a plurality of events occurring with respect to the one or more medical devices; and
   data indicative of one or more environmental parameters associated with the one or more medical devices;
(e) providing data indicative of the tracked performance metric to the analysis model; and
(f) updating the analysis model by further training it using the data provided in steps (d) and (e).

The data indicative of one or more environmental parameters associated with the one or more medical devices may be received from one or more of: temperature sensors; humidity sensors; vibration sensors; and light sensors.

The data indicative of a plurality of events occurring with respect to the one or more medical devices may include data obtained from sources including one or more of: laboratory instruments, software configured to receive information from laboratory instruments, point-of-care devices, laboratory middleware, a hospital information system, software configured to receive information from a hospital information system, a transport layer, and a centralized event log.

In a second aspect, embodiments of the present disclosure provide a computer-implemented method of monitoring a medical device comprising:
receiving and processing, by a data processing module, data related to one or more processes associated with the medical device;
displaying, by a user terminal, one or more tracked performance metrics associated with the or each process;
receiving, from a user via the user terminal, a selected time in a history of the tracked performance metric and providing the selected time to the data processing module;
deriving from the received and processed data, by the data processing module, at least one factor which has contributed to the value of the tracked performance metric at the selected time, the or each factor comprising an event which has taken place with respect to the respective process; and
output to the user, via the user terminal, the or each derived factor.

The method of the second aspect may include any one, or any combination insofar as they are compatible, of the optional features of the system of the first aspect.

In a third aspect, embodiments of the present disclosure provide a medical device monitoring engine, comprising:
a data processing module, configured to receive and process data related to one or more processes associated with one or more medical devices; and
a user interface module, configured to display one or more tracked performance metrics associated with the or each process, and to receive, from the user, a selected time in a history of the tracked performance metric and to provide the selected time to the data processing module;
wherein the data processing module is configured, in response to receiving the selected time, to:
   derive from the received and processed data at least one factor which has contributed to the value of the tracked performance metric at the selected time, the or each factor comprising an event which has taken place with respect to the respective process, and
   output to the user, via the user interface module, the or each derived factor.

The medical device monitoring engine may be installed within a user terminal, a server, a cloud-based computing server, a laboratory middleware, or an intermediary device connectable to a laboratory middleware and to a user terminal.

The medical device monitoring engine may be implemented as computer software, and may be stored in a non-transitory computer readable storage medium containing machine executable instructions which, when executed on a processor, implement the data processing module and the user interface module. By implement, it may be meant that software providing the functions described is installed and operated.

The medical device monitoring engine of the third aspect may include any one, or any combination insofar as they are compatible, of the optional features of the system of the first aspect.

The disclosure includes the combination of the aspects and features described except where such a combination is clearly impermissible or expressly avoided.

In a further aspect, embodiments of the disclosure provide a non-transitory computer readable storage medium containing machine executable instructions which, when executed on one or more processors, cause the one or more processors to perform the method of the second aspect.

Further aspects of the present disclosure provide: a computer program comprising code which, when run on a computer, causes the computer to perform the method of the first aspect; a computer readable medium storing a computer program comprising code which, when run on a computer, causes the computer to perform the method of the second aspect; and a computer system programmed to perform the method of the second aspect.

### Brief Description of the Drawings

Embodiments of the disclosure will now be described by way of example with reference to the accompanying drawings in which:
- Figure 1: is a network diagram including a medical device management system;
- Figure 2: is a diagram showing components of the medical device management system;
- Figure 3: is an example of a user terminal; and
- Figure 4: is a flow diagram of a computer-implemented method.

### Detailed Description and Further Optional Features

Aspects and embodiments of the present disclosure will now be discussed with reference to the accompanying figures. Further aspects and embodiments will be apparent to those skilled in the art.

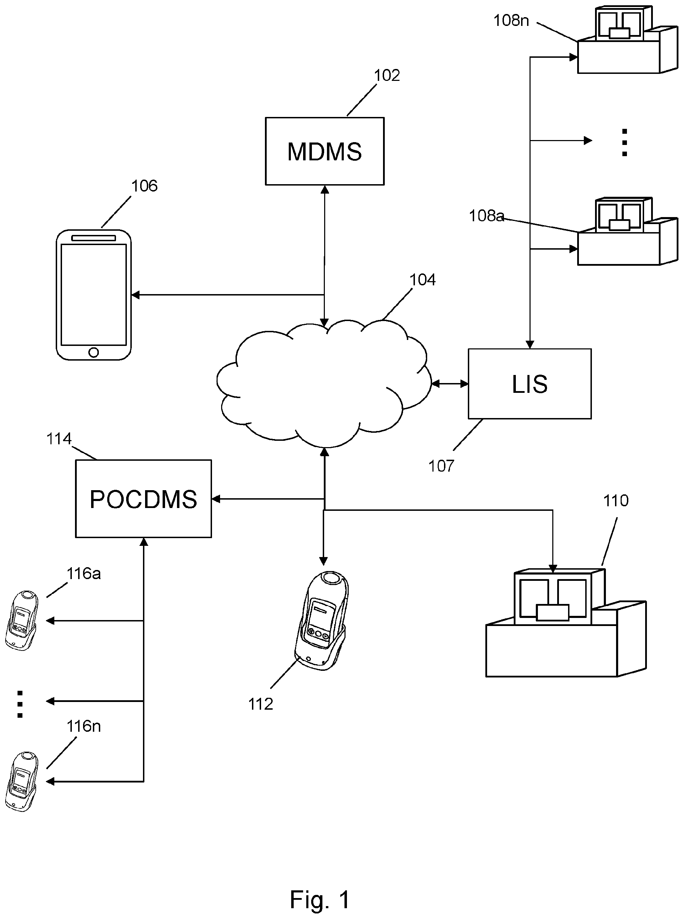

Figure 1 is a network diagram including a medical device management system 102. The medical device management system is connected via network 140 to a number of devices and components. In this example, the medical device management system is connected via the network to a user terminal 106. The medical device management system provides data to and receives data from the user terminal. For example, the user terminal may request information on a tracked performance metric (e.g. turnaround time). The medical device management system locates this and sends it to the user terminal for display. The user, via the user terminal, then selects a time in a history of the tracked performance metric and this selected time is provided to the medical device management system.

The medical device management system then uses previously received and processed data to identify one or more factors which have contributed to the value of the tracked performance metric at the selected time. The or each factor identifies or comprises an event which has taken place with respect to the respective process. The medical device management system then provides this factor to the user via the user terminal.

The medical device management system is also connected to various sources of data, e.g. process data which describes one or more processes associated with one or more medical devices. In this example, the medical device management system 102 is connected to a laboratory information system 107 (also referred to as a laboratory management system, or laboratory middleware). The laboratory information system is connected to a plurality of laboratory instruments 108a to 108n, each of which can perform one or more laboratory processes (e.g. tests). The instruments provide data back to the LIS, which in turn provides data via the network to the medical device management system 102. The data includes, for example, the direct result of various tests. But it can also include various operating parameters and/or conditions of the or each laboratory instrument. Additionally, or alternatively, the LIS may be configured to derive such parameters or conditions based on other information it has available to it from the instruments.

The medical device management system 102 may also be directly connected to one or more laboratory instrument 110, that is instead of via an LIS. The operation is essentially the same, and the medical device management system may derive various parameters and/or conditions of the laboratory instrument based on information it has available to it.

The medical device management system 102 is also, in this example, connected to a point-of-care device management system (POCDMS) 114. The POCDMS can be, for example, a cobas infinity solution which monitors and receives data from a plurality of point-of-care devices 116a - 116n. The point-of-care devices can each perform one or more clinical processes (e.g. tests). The point-of-care devices provide data back to the POCDMS, which in turn provides data via the network to the medical device management system 102. The data includes, for example, the direct result of various tests. But it can also include various operating parameters and/or conditions of the or each point-of-care device. Additionally, or alternative, the POCDMS may be configured to derive such parameters or conditions based on other information it has available to it from the point-of-care devices.

The medical device management system 102 may also be directly connected to one or more point-of-care devices 112, that is instead of via a POCDMS. The operation is essentially the same, and the medical device management system may derive various parameters and/or conditions of the laboratory instrument based on information it has available to it.

In both situations, the data received by the medical device management system may include data indicative of one or more environmental parameters associated with the one or more medical devices. This can include data from: temperature sensors; humidity sensors; vibration sensors; and light sensors. The data indicative of a plurality of events occurring with respect to the one or more medical devices, as received by the medical device management system, may include data obtained from sources including one or more of: a hospital information system, software configured to receive information from a hospital information system, a transport layer, and a centralized event log.

Two examples are considered. In the first, the medical device management system 102 is connected to and receives data from one or more laboratory instruments. The user, via the user terminal, requests that the tracked performance metric "turnaround time" is displayed. The user terminal fetches from, for example, the medical device management system 102 (although this data may in fact be cached on the user terminal, or stored on cloud storage, etc.) historical values of turnaround time for the instrument(s) or laboratory containing the instrument(s) and displays it to the user. The user then selects a point in time where the turnaround time peaked, this representing an undesirable situation for the laboratory where the turnaround time was unacceptably high. The selected point in time is then sent to the medical device management system where one or more factors are derived (e.g. using an analysis model which may be machine learning based) which have contributed to the value of the turnaround time at the selected time. In this example, it has been ascertained that a reagent in one or more instruments ran out and that the only technician certified to restock this reagent was not scheduled to be working at that time. The medical device management system may, in some circumstances, use the prediction module (discussed below) to predict the impact of various changes to the laboratory and offer them to the user to remedy the impact in performance. In this example, the medical device management system may recommend that the technician's working hours be rescheduled to overlap with the point in time where the reagent is most likely to run out and so they should be available to restock it.

In a second example, the medical device management system 102 is connected to and receives data from one or more point-of-care devices. The user, via the user terminal, requests that the tracked performance metric "device uptime" is displayed e.g. as measured by the percentage of the previous hour period that the device was available for use. The user terminal fetches from, for example, the medical device management system 102 (although this data may in fact be cached on the user terminal, or stored on cloud storage, etc.) historical values of device uptime for the point-of-care device(s) and displays it to the user. The user then selects a point in time where the device uptime was at its lowest, this representing an undesirable situation where the device availability was unacceptably low. The selected point in time is then sent to the medical device management system where one or more factors are derived (e.g. using an analysis model which may be machine learning based) which have contributed to the value of the device uptime at the selected time. In this example, it has been ascertained that the point-of-care device(s) ran software or firmware updates during busy hours for the hospital in which the point-of-care device(s) are used. The medical device management system may, in some circumstances, use the prediction module to predict the impact of various changes to the point-of-care devices and offer them to the user to remedy the impact in performance. In this example, the medical device management system may recommend that the point-of-care device(s) are updated at a different time of day when they are less likely to be needed, or may stagger the timing of updates across a plurality of point-of-care devices.

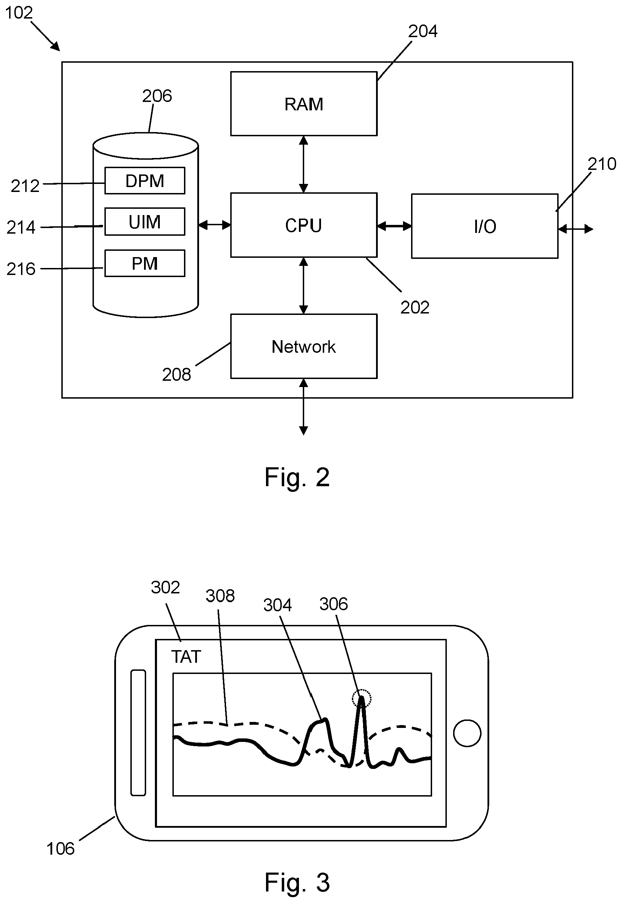

Figure 2 is a diagram showing components of the medical device management system 102. The medical device management system 102 in this example includes the following components: central processing unit (CPU) 202, random access memory (RAM) 204, permanent storage medium 206, network interface 208, and input/output interface 210. The network interface allows the medical device management system to communicate with physically external components, e.g. via network 104 as discussed above. The input/output interface 210 allows for connection, for example, to user input device such as a keyboard as well as display means.

The permeant storage medium 206 contains executable code which, when loaded into the RAM 204 and processed on processor 202 implements a data processing module 212 as discussed previously, user interface module 212, and a prediction module 216. The data processing module 212 is the component of the medical device monitoring system configured to receive and process data related to one or more processes associated with the one or more medical devices. The user interface module 214 is configured to receive, e.g. from the user terminal, the selected time and provide it to the data processing module. The data processing module then being configured to derive from the received and processed data the at least one factor. The user interface module 214 then provides this factor to the user terminal for presentation to the user. The user interface module may also be configured to track the current (e.g. live) value of the tracked performance metric, and to alert the user (e.g. via the user terminal) when the tracked performance metric deviates from a predefined acceptable range. The user interface module may also be configured to receive, from the user terminal, a definition of a performance metric to be tracked. This definition can then be provided to the data processing module for it to then receive and process data relating to the defined performance metric. The prediction module 216 may receive a user specified factor and predict an impact on the tracked performance metric. It can also be used to predict what the current value of the tracked performance metric ought to be (as a prediction value), and then alert the user via the user interface module 214 if the current value has deviated significantly from the predicted value. The combination of at least the data processing module 212 and user interface module 214 provides a medical device monitoring engine which can be installed and operated on any given piece of computing hardware.

The data processing module 212 may include an analysis model, which may be a machine-learning model. The machine-learning model may have been trained by, in an initial phase: (a) receiving input data comprising one or more of: data indicative of a plurality of events occurring with respect to the one or more medical devices; and data indicative of one or more environmental parameters associated with the one or more medical devices; (b) receiving data indicative of the tracked performance metric; and (c) training the machine-learning model using the input data as an input and the data indicative of the performance of the tracked performance metric as a desired output. This can allow the trained machine-learning model to then relate new input data to the tracked performance metric.

Steps (a) - (c) as discussed above may be performed on historical input data and historical data indicative of the tracked performance metric. The analysis model may be continuously retrained by: (d) providing additional input data to the analysis model comprising one or more of: data indicative of a plurality of events occurring with respect to the one or more medical devices; and data indicative of one or more environmental parameters associated with the one or more medical devices; (e) providing data indicative of the tracked performance metric to the analysis model; and (f) updating the analysis model by further training it using the data provided in steps (d) and (e).

In examples where the medical device monitoring system 102 is operating on the user terminal, the user interface module may provide the derived factor to the I/O interface 210 for display on a connected display unit. Where the medical device monitoring system 102 is operating on a device physically distinct from the user terminal, the factor may be provided to the network interface 208 for transmission over the network to the user terminal.

Figure 3 is an example of a user terminal 106. It displays via a display a label 302 for the tracked performance metric, in this example turnaround time (TAT). The display shows the tracked performance metric as a line 304 with the x-axis representing time, and the y-axis representing the value of the tracked performance metric. The user selects, e.g. via a touch-interface or other user input device, a point in time 306. This selected time is then provided to the medical device management system 102 in the manner discussed above. Also shown is an optional benchmarking line 308, which shows the user a benchmark tracked metric value for comparison with the tracked metric value. Once the user terminal 106 receives the one or more factors which contributed to the value of the tracked performance metric at the selected time, the display of the user terminal 106 changes to show the user the derived factors.

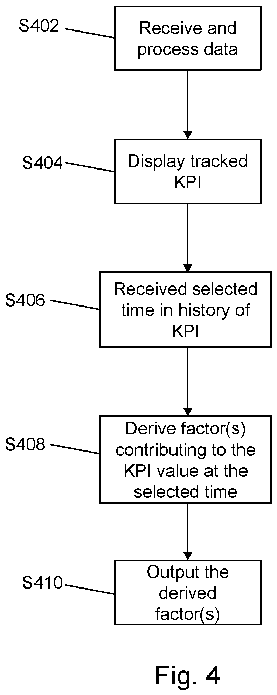

Figure 4 is a flowchart illustrating a method according to the disclosure. In a first step, S402, data related to one or more processes associated with one or more medical devices is received and processed. Next, in step S404, a tracked performance metric (also referred to as a key performance indicator or KPI) is displayed to the user. In step S406, a selected time in the history of the tracked performance metric is received from the user. Using this, factor(s) contributing to the value of the tracked performance metric at the selected time are derived in step S408. These derived factors are then outputted to the user in step S410.

The term 'laboratory instrument' as used herein encompasses any apparatus or apparatus component operable to execute one or more processing steps / workflow steps on one or more biological samples and/or one or more reagents. The expression 'processing steps' thereby refers to physically executed processing steps such as centrifugation, aliquotation, sample analysis and the like. The term 'instrument' covers pre-analytical instruments, post-analytical instruments and also analytical instruments.

The term 'analyzer' / 'analytical instrument' as used herein encompasses any apparatus or apparatus component configured to obtain a measurement value. An analyzer is operable to determine via various chemical, biological, physical, optical or other technical procedures a parameter value of the sample or a component thereof. An analyzer may be operable to measure said parameter of the sample or of at least one analyte and return the obtained measurement value. The list of possible analysis results returned by the analyzer comprises, without limitation, concentrations of the analyte in the sample, a digital (yes or no) result indicating the existence of the analyte in the sample (corresponding to a concentration above the detection level), optical parameters, DNA or RNA sequences, data obtained from mass spectrometry of proteins or metabolites and physical or chemical parameters of various types. An analytical instrument may comprise units assisting with the pipetting, dosing, and mixing of samples and/or reagents. The analyzer may comprise a reagent holding unit for holding reagents to perform the assays. Reagents may be arranged for example in the form of containers or cassettes containing individual reagents or group of reagents, placed in appropriate receptacles or positions within a storage compartment or conveyor. It may comprise a consumable feeding unit. The analyzer may comprise a process and detection system whose workflow is optimized for certain types of analysis. Examples of such analyzers are clinical chemistry analyzers, coagulation chemistry analyzers, immunochemistry analyzers, urine analyzers, nucleic acid analyzers, used to detect the result of chemical or biological reactions or to monitor the progress of chemical or biological reactions.

The term 'pre-analytical instrument' as used herein encompasses any apparatus or apparatus component that is configured to perform one or more pre-analytical processing steps / workflow steps comprising - but not limited to - centrifugation, resuspension (e.g. by mixing or vortexing), capping, decapping, recapping, sorting, tube type identification, sample quality determination and/or aliquotation steps. Said processing steps may also comprise adding chemicals or buffers to a sample, concentrating a sample, incubating a sample, and the like.

The term 'post-analytical instrument' as used herein encompasses any apparatus or apparatus component that is configured to perform one or more post-analytical processing steps / workflow steps comprising - but not limited to - sample unloading, transport, recapping, decapping, temporary storage / buffering, archiving (refrigerated or not), retrieval and/ or disposal.

The term point-of-care device as used herein encompasses any analyzer used in a point-of-care environment, such as (but not limited to) blood glucose testing, coagulation testing, blood gas and electrolytes analysis, urinalysis, cardiac markers analysis, hemoglobin diagnostics, infectious disease testing, cholesterol screening or nucleic acid testing NAT. Results may be viewed directly on the POC analyzer/device(s) or may be sent to a POCDMS and displayed in a Laboratory Information System with central lab results, or alongside imaging results in a Hospital Information System.

The systems and methods of the above embodiments may be implemented in a computer system (in particular in computer hardware or in computer software) in addition to the structural components and user interactions described.

The term "computer system" includes the hardware, software and data storage devices for embodying a system or carrying out a method according to the above described embodiments. For example, a computer system may comprise a central processing unit (CPU), input means, output means and data storage. The computer system may have a monitor to provide a visual output display. The data storage may comprise RAM, disk drives or other computer readable media. The computer system may include a plurality of computing devices connected by a network and able to communicate with each other over that network.

The methods of the above embodiments may be provided as computer programs or as computer program products or computer readable media carrying a computer program which is arranged, when run on a computer, to perform the method(s) described above.

The term "computer readable media" includes, without limitation, any non-transitory medium or media which can be read and accessed directly by a computer or computer system. The media can include, but are not limited to, magnetic storage media such as floppy discs, hard disc storage media and magnetic tape; optical storage media such as optical discs or CD-ROMs; electrical storage media such as memory, including RAM, ROM and flash memory; and hybrids and combinations of the above such as magnetic/optical storage media.

While the disclosure has been described in conjunction with the exemplary embodiments described above, many equivalent modifications and variations will be apparent to those skilled in the art when given this disclosure. Accordingly, the exemplary embodiments of the disclosure set forth above are considered to be illustrative and not limiting. Various changes to the described embodiments may be made without departing from the spirit and scope of the disclosure.

In particular, although the methods of the above embodiments have been described as being implemented on the systems of the embodiments described, the methods and systems of the present disclosure need not be implemented in conjunction with each other, but can be implemented on alternative systems or using alternative methods respectively.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the disclosure in diverse forms thereof.

While the disclosure has been described in conjunction with the exemplary embodiments described above, many equivalent modifications and variations will be apparent to those skilled in the art when given this disclosure. Accordingly, the exemplary embodiments of the disclosure set forth above are considered to be illustrative and not limiting. Various changes to the described embodiments may be made without departing from the spirit and scope of the disclosure.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

### Reference numerals

- 102: Medical device monitoring system
- 104: Network
- 106: User terminal
- 107: Laboratory information system
- 108a-n: Laboratory instrument
- 110: Laboratory instrument
- 112: Point-of-care device
- 114: Point-of-care device management system
- 116a-n: Point-of-care device
- 202: CPU
- 204: RAM
- 206: Permeant storage
- 208: Network interface
- 210: I/O interface
- 212: Data processing module
- 214: User interface module
- 302: Tracked metric label
- 304: Tracked metric line
- 306: Selected time
- 308: Benchmark metric line

## Claims

1. A medical device monitoring system comprising:
a data processing module, configured to receive and process data related to one or more processes associated with one or more medical devices; and
a user terminal, configured to display one or more tracked performance metrics associated with the or each process, and to receive, from a user, a selected time in a history of the tracked performance metric and to provide the selected time to the data processing module;
wherein the data processing module is configured, in response to receiving the selected time, to:
derive from the received and processed data at least one factor which has contributed to the value of the tracked performance metric at the selected time, the or each factor comprising an event which has taken place with respect to the respective process, and
output to the user, via the user terminal, the or each derived factor.

2. The medical device monitoring system of claim 1, wherein the data relates to performance of one or more of: a work area in a laboratory; a testing process performed in a laboratory; a point-of-care device; and a laboratory instrument used in a laboratory.

3. The medical device monitoring system of claim 1 or 2, wherein one of the data processing module and the user terminal is configured to provide alerts to a user when the tracked performance metric deviates from a predefined acceptable value range.

4. The medical device monitoring system of any preceding claim, further comprising a prediction module configured to predict an impact a user specified factor has on the tracked performance metric.

5. The medical device monitoring system of any preceding claim, wherein the user terminal is one of: a smart phone, a tablet, a laptop computer, and a desktop computer.

6. The medical device monitoring system of any preceding claim, wherein the tracked performance metric is one of: a sample turnaround time, a late sample tracking value, connection status, sample workload, laboratory instrument throughput, reagent capacity, a quality control value, and a point-of-care device uptime.

7. The medical device monitoring system of any preceding claim, wherein the user terminal is configured to receive, from the user, a definition of a performance metric to be tracked, and wherein the data processing module is configured to then receive and process data relating to the defined performance metric.

8. The medical device monitoring system of any preceding claim, wherein the user terminal is further configured to display to the user a benchmark performance metric associated with the or each process for comparison with the tracked performance metric.

9. The medical device monitoring system of any preceding claim, wherein deriving the at least one factor includes using an analysis model which relates data pertaining to the process to a value of the performance metric.

10. The medical device monitoring system of claim 9, wherein the analysis model is a machine-learning model which has been trained by, in an initial training phase:
(a) receiving input data comprising one or more of:
data indicative of a plurality of events occurring with respect to the one or more medical devices; and
data indicative of one or more environmental parameters associated with the one or more medical devices;
(b) receiving data indicative of the tracked performance metric; and
(c) training the machine-learning model using the input data as an input and the data indicative of the performance of the tracked performance metric as a desired output, whereby the trained machine-learning model is able to relate new input data to the tracked performance metric.

11. The medical device monitoring system of claim 10, wherein steps (a) - (c) are performed on historical input data and historical data indicative of the tracked performance metric; and
the analysis model is continuously retrained by:
(d) providing additional input data to the analysis model comprising one or more of:
data indicative of a plurality of events occurring with respect to the one or more medical devices; and
data indicative of one or more environmental parameters associated with the one or more medical devices;
(e) providing data indicative of the tracked performance metric to the analysis model;
and
(f) updating the analysis model by further training it using the data provided in steps (d) and (e).

12. The medical device monitoring system of claim 10 or 11, wherein:
the data indicative of one or more environmental parameters associated with the one or more medical devices is received from one or more of: temperature sensors; humidity sensors; vibration sensors; and light sensors.

13. The medical device monitoring system of any of claims 10 - 12, wherein:
the data indicative of a plurality of events occurring with respect to the one or more medical devices includes data obtained from sources including one or more of: laboratory instruments, software configured to receive information from laboratory instruments, point-of-care devices, laboratory middleware, a hospital information system, software configured to receive information from a hospital information system, a transport layer, and a centralized event log.

14. A computer-implemented method of monitoring a medical device comprising:
receiving and processing, by a data processing module, data related to one or more processes associated with the medical device;
displaying, by a user terminal, one or more tracked performance metrics associated with the or each process;
receiving, from a user via the user terminal, a selected time in a history of the tracked performance metric and providing the selected time to the data processing module;
deriving from the received and processed data, by the data processing module, at least one factor which has contributed to the value of the tracked performance metric at the selected time, the or each factor comprising an event which has taken place with respect to the respective process; and
output to the user, via the user terminal, the or each derived factor.

15. A medical device monitoring engine, comprising:
a data processing module, configured to receive and process data related to one or more processes associated with one or more medical devices; and
a user interface module, configured to display one or more tracked performance metrics associated with the or each process, and to receive, from the user, a selected time in a history of the tracked performance metric and to provide the selected time to the data processing module;
wherein the data processing module is configured, in response to receiving the selected time, to:
derive from the received and processed data at least one factor which has contributed to the value of the tracked performance metric at the selected time, the or each factor comprising an event which has taken place with respect to the respective process, and
output to the user, via the user interface module, the or each derived factor.
